# EUROPEAN PATENT APPLICATION

(11) **EP 3 015 110 A1**
(43) Date of publication of application: **04.05.2016**
(21) Application number: 15191583.2
(22) Date of filing: 27.10.2015
(51) Int. Cl.: A61K 35/744, A61K 31/198, A61P 11/00, A61P 11/12

(54) **BIOLOGICAL BARRIER WITH CYSTEIN FOR THE USE IN THE TREATMENT OF NASO-PHARYNGO-TUBAL INFECTIONS**

(30) Priority: 28.10.2014 IT RM20140611
(71) Applicant: D.M.G. Italia Srl, 00071 Pomezia (Rome) (IT)
(72) Inventor: Mercuri, Luigi, I-00071 Pomezia (Rome) (IT); Tiberi, Licia, I-00071 Pomezia (Rome) (IT)
(74) Representative: Sarpi, Maurizio

(57) **Abstract**

Association of *Streptococcus salivarius* DSM 23307 and cysteine as the active ingredient to reduce the surface tension of the nasal mucus and to restore the biological protective barrier function against pathogens for use in the treatment and prevention of naso-pharyngo-tubal infections. Described are also compositions that comprises it, and the kit which makes easier the administration of the composition in the subjects in the need thereof.

## Description

### Scope of the invention

The present invention relates to prophylactic and therapeutic treatment of infections of the naso-pharyngeal district (frequent colds, sinusitis, tonsillitis, diseases related to adenoid hypertrophy, respiratory allergies) and, in particular, of the middle ear (otitis, tubal-tympanitis, recurrent acute otitis media AOM).

### Background Art

Many of ENT (ear, nose and throat) diseases originate from a fungal or bacterial infection of the upper respiratory tract; examples of these infections are the otitis, sinusitis and/or nasal polyposis.

The inflammation of nasopharynx and adenoidal hypertrophy, if neglected or poorly treated, leads to complications in the middle ear. In particular, among the various bacterial pathologies ENT diseases have been found most frequently in children: in the first three years of life almost all children run into at least one episode of AOM and about a third have three or more episodes.

One of the causes of acute otitis media is the impaired ventilation of the middle ear resulting in a tubal obstruction, linked to the inflammation associated to a viral infection of the upper respiratory tract. Such condition facilitates the ascent of respiratory pathogens (*Streptococcus pneumoniae, Haemophilus influenzae* and *Moraxella catharralis*) that colonize the nasopharynx spreading to the tympanic cavity.

Usually the treatment of these pathologies requires the use of topical/oral anti-inflammatory drugs or antibiotics. It is known that the excessive use of antibiotics, especially in Western countries, has resulted in the selection of strains resistant to the treatments, some of which even in all classes of antibiotics currently available. The increasing difficulty in finding new effective antibiotic molecules directed research interest towards alternative therapeutic approaches, which potentially offer greater specificity and safety, lack of drug interactions and ability to leverage complementary mode of action than antibiotics, drastically reducing the risk of the development of further resistance.

ENT diseases involve a significant commitment of care because they require at least a medical examination, prescription of antipyretic medicaments and often antibiotics. In addition to direct costs, the AOM involves high indirect costs due to absence from work of at least one parent and a negative impact on life quality of the children and their families. Such negative effects are multiplied in the case of recurrent AOM.

Therefore, treatment and prevention of ENT diseases in general, and of AOM in particular, is now a primary focus of pediatric medical care.

As the AOM is favoured by a large number of factors, usually following a viral or a bacterial infection of the upper respiratory tract, attempts to prevent the disease have to be mainly based on reduction of risk factors, incidence of viral respiratory infections and bacterial colonization of the upper respiratory tract.

Several approaches to solve the problem are available, but all, generally of limited effectiveness. Environmental prophylaxis, intended as corrective action on the factors favouring the relapse, is the first step in the prevention of ENT diseases, but not optimally effective. Staying in their own home rather than attending the nursery can avoid two out of five episodes in the case of children with ear disease. The use of appropriate hygiene measures, such as thorough hand washing or using alcoholic detergent solutions, in kindergartens results in a 25% reduction of AOM episodes. The use of the pacifier only in falling asleep step reduces the risk of AOM of 29%.

For a long time chemoprophylaxis has been the easier preventive medical approach. In the '90s it has been shown to be effective in preventing the recurrence of episodes, reducing the risk of occurrence of new episodes up to 60%. However, antibiotic prophylaxis requires good compliance of the child and the family, not always easily achieved for long periods of time, at least three months. Moreover, in recent years the use of antibiotic prophylaxis has been heavily debated, and consequently limited to highly selected cases, due to the potential risk of bacterial resistance.

During the circulation of influenza viruses the administration of the vaccine reduces the incidence of the disease in healthy children without recurrent AOM. The results vary depending on the type of vaccine and the age of the children: the vaccines of attenuated and alive virus determine the best results (reduction of up to 90%) than those obtained with the inactivated virus vaccines (reduction of up to 50%), especially in children under 18 months. In children with a history of recurrent AOM, the inactivated vaccine is able to reduce by about 33% recurrences, with greater effectiveness in cases of uncomplicated AOM (47%) than those complicated by spontaneous perforation and otorrhea (20%).

Finally, another prevention approach resulted to be very valid is based on modification of the bacterial flora. Colonization by respiratory pathogens is one of the main risk factor for AOM in the first years of life and an increase in bacterial load in nasopharyngeal district is associated with the occurrence of AOM. Concurrently to the increase of respiratory pathogens, a reduction of non-pathogenic and protective microorganisms, such as *Streptococcus α-hemoliticus,* has been observed. It has been noted that the absence and/or the low bacterial load of *Streptococcus α-hemoliticus* is closely related to the occurrence of acute episodes. The modification of the nasopharyngeal bacterial microecology could therefore constitute an optimal way to yield effective prevention, since directly acting on the cause of infectious disease of the middle ear.

Recently, several clinical trials have shown that the administration of streptococci such as *Streptococcus mitis, Streptococcus sanguinis, Streptococcus oralis* in the form of sprays in patients with AOM interferes and/or inhibits the growth of pathogenic microorganisms responsible for the disease.

A probiotic strain specifically used for oral hygiene and against halitosis, *S. salivarius* K12, has been orally administrated to a group of children suffering from OMA through a composition comprising; the study has revealed that in a small percentage of treated patients the strain *S. salivarius* K12 can colonize the upper respiratory improving the symptoms of the disease [Power D.A. et al. Preliminary investigations of the colonisation of upper respiratory tract tissues of infants using a paediatric formulation of the oral probiotic Streptococcus salivarius K12. Eur J Clin Microbiol Infect Dis. 2008, 27: 1261-1263].

The applicants of the present application have isolated from the nasopharyngeal tract of a healthy volunteer, a new bacterial strain belonging to the species of *Streptococcus salivarius,* deposited at the Institut Deutsche Sammlung von Mikroorganismen und Zellkulturen (DSMZ) with the deposit number DSM 23307. Strain characterization studies have shown that *S. salivarius* DSM 23307 has a strong inhibitory activity *in vitro* against *S. Pneumonia.* The strain is stable under different growth conditions, it has an inhibitory activity against virulent and multi-resistant to antibiotics serotypes causing infections such as the invasive strains of *S. pneumoniae* 19 A. Furthermore, the newly identified strains has inhibitory activity against *S. pyrogenes* M-1 type, high adhesion to HEp-2 cells (epithelial cells from human laryngeal carcinoma) up to 57%, absence of virulence genes (WO 2011/125086 and Santagati et al. Bacteriocin-producing oral streptococci and inhibition of respiratory pathogens. FEMS Immunol Med Microbiol. 2012.65: 23-31).

Due to its features the strain *S. salivarius* DSM23307 is particularly suitable for use in the treatment of bacterial infections and/or fungal infections of the upper portions of the respiratory system. This bacterial strain can be administered by means of appropriate pharmaceutical compositions, and is able to colonize the respiratory tract by competing with pathogenic species. The patent application WO 2011/125086 describes the original bacterial strain *S. salivarius* DSM 23307, and the compositions that comprise it, for the use in the treatment of infections of the middle ear, often resulting from infection of the upper respiratory tract (such as a cold).

Hence, the use of compositions comprising certain strains of *S. salivarius* for the treatment of otitis media is known from the patent documents WO 2004/072272 and WO 2011/125086.

In particular, in the international patent application WO 2011/125086, the Applicant has demonstrated the effectiveness of the strain *S. salivarius* DSM 23307 as oral probiotic able to inhibit the action of respiratory pathogens. Pharmaceutical compositions comprising said microorganism represent a useful therapeutic tool in the treatment of infections of the upper respiratory tract. The ability to interfere with the growth of pathogenic microorganisms infections of the upper respiratory tract provides an important means of defence against pathogens. When the indigenous bacterial flora is present on the surface to be colonized, occupying space and competing for sites of cell adhesion, effectively it prevents access to the pathogen, and the adhesion, to the cell surface, thus blocking the colonization, growth and subsequent infection by pathogenic strains.

Following the identification of the bacterial strain *S. salivarius* DSM 23307, its therapeutic properties and use in the pharmaceutical compositions for the treatment of respiratory tract infections, it is strongly felt the development of particular associations of ingredients that in combination with said bacterial strain, provide the composition including them with an improved technical effect useful in the treatment and prevention of bacterial or viral etiology ENT diseases.

It has now surprisingly been found that the association of the strain *S. salivarius* DSM 23307 and cysteine, which is used as a mucolytic agent, provides a synergistic effect such that the composition comprising the association has an efficacy superior to that obtained by the use of compositions based on the bacterial strain only, performing a useful action effective in the treatment of naso-pharyngeal-tubal pathologies. This has been experimentally demonstrated by comparing patients undergoing treatment with the individual active constituents and with a composition of the placebo.

### SUMMARY OF THE INVENTION

In a first aspect the invention relates to the association of the bacterial strain *Streptococcus salivarius* DSM 23307 and cysteine having a local antibacterial and anti-inflammatory activity and is used to treat the inflammations and infections of the naso-pharyngo-tubal district.

In a second aspect the invention relates to the composition comprising the association of the bacterial strain *Streptococcus salivarius* DSM 23307 and cysteine having the ability to act as local biological barrier in the treatment of inflammations and infections of the naso-pharyngo-tubal district.

In a third aspect the invention provides a product consisting of the composition comprising the association of the bacterial strain *S. salivarius* DSM 23307 and cysteine both reconstituted in a suitable solvent so as to be deliverable through an atomizer spray bottle in the naso-pharyngo-tubal district.

In a fourth aspect the invention provides a user friendly kit allowing easier use of the composition according to the invention by the end user.

In a further aspect the invention provides the association of the bacterial strain *Streptococcus salivarius* DSM 23307 and cysteine, and the composition comprising the association of the bacterial strain *Streptococcus salivarius* DSM 23307 and cysteine for the use in the treatment of inflammations and infections of the naso-pharyngo-tubal district.

The advantages, features and the modes of employ of the present invention will become apparent from the following detailed description of some embodiments thereof, given by way of example and not of limitation.

### DETAILED DESCRIPTION OF THE INVENTION

The components of the association according to the invention are the bacterial strain *Streptococcus salivarius* DSM 23307 and cysteine.

Cysteine is a polar sulfur-containing amino acid, chemically is 2-amino-3-sulfhydrylpropanoic acid. It is a constituent of many proteins and its -SH group can be easily oxidized by a number of oxidizing agents, including molecular oxygen. In an oxidizing environment two -SH groups can bind a water molecule and form a disulfide bridge (-SS-). These bonds allow two cysteine units placed at different points of the polypeptide chain, or in two different polypeptide chains, to bind to each other, making the stable tertiary structure (three-dimensional) of the protein.

Cysteine and the N-acetyl derivative thereof, N-acetyl-cysteine (NAC), have many therapeutic applications, including that as mucolytic agent. The mucolytic activity of cysteine and N-acetyl-cysteine is due to its ability to reduce the characteristic disulfide bonds of the mucoproteins, by the free sulphydryl group interacting with the bonds responsible for the aggregation of proteins, and hence the high viscosity of the mucus. As a result of the interchange sulfhydryl-disulfide, glycoprotein molecules are broken down into smaller units with lower viscosity, and the modification of the mucus rheological properties results in easier expectoration. Generally, in pharmacology the acetylated form of the most common cysteine finds major use for its greater stability and bioavailability, compared to the non-acetylated form. Following oral administration, NAC is rapidly absorbed, peaking up blood 2-3 hours after ingestion, with a half-life of about 6 hours; N-acetyl cysteine smoothly enters the cell, where it is deacetylated regenerating the L-Cysteine.

However, the mucolytic activity of the cysteine is greater than the acetylated form thereof, and therefore its direct use in the molecules intended for medical use, although less stable, has to be considered preferable. The Applicant has experimentally shown that in the particular association according to the invention, with a combined action, the association of streptococcus and cysteine constitutes an active principle with increased ability to break the disulfide bridges of the glycoproteins of the mucosal secretions by facilitating the elimination of this latter and simultaneously to reconstitute the biological barrier protective against pathogens for use in the treatment and prevention of naso-pharyngo-tubal infections.

The pharmaceutical compositions comprising the association of streptococcal and cysteine may further comprise one or more pharmaceutically acceptable vehicles, diluents and/or excipients, and optionally, the compositions may also comprise further active ingredients.

In the association according to the invention cysteine is provided as cysteine hydrochloride.

According to the invention cysteine hydrochloride and bacteria are provided in lyophilized form. The preparation of the composition of the invention can then be implemented by means of fermentation and lyophilization of the bacterial culture, lyophilisation of cysteine hydrochloride, and the subsequent mixing of the lyophilized and dissolved in the suitable solvent. According to the invention the suitable solvent is an isotonic saline solution buffered at pH 7.0 comprising the PEG/PPG copolymer (INCI: Bis-PEG13 Methoxy PEG-438/PPG-110 SMDI Copolymer), and flavourings.

The value of pH equal to 7.0 and the osmolarity of 300 mOsm of the saline solution, wherein the lyophilized powders are dissolved is achieved by the use of the buffering system consisting of monobasic sodium phosphate/dibasic sodium phosphate and sodium chloride. According to the invention also other buffering systems known to the expert of the field can be used.

In a first embodiment of the invention *S. salivarius* micro-organisms and cysteine hydrochloride are lyophilized and supplied separately to the end user of the therapeutic composition who before using dissolves them in isotonic saline solution buffered at pH 7.0 according to the invention at the time of use. The solution comprising the association of the microorganism and cysteine so obtained is stable and retains its effectiveness for 5 days, which is the time range by which the composition should be administered to the patient.

Alternatively, in a second embodiment of the invention cysteine hydrochloride is supplied in lyophilized form together with the bacterium, the mixture so obtained must be reconstituted in the solvent by the recipient of the treatment.

Providing microorganism and cysteine together in lyophilized form makes the preparation of the drug easier for the end user by increasing the compliance of the patient.

The preparation of the composition comprising lyophilized association of microorganisms and cysteine hydrochloride is implemented through standard methods of fermentation and freeze-drying known to the skilled of the field before using.

The final reconstituted suspension contains *S. salivarius* DSM 23307 in an amount ranging between 3 x 10⁵ and 3 x 10¹⁴ cfu (colony forming units) per gram of the composition in a volume of 10-50 ml, preferably 1-5x10¹¹ cfu of per gram of composition in a 10-15 ml volume.

Said compositions may also include carriers and vehicles suitable to increase the bioavailability, stability and tolerability of the microorganism and to further improve the adhesion of the microorganism on the mucosal surface such as the polymer PEG/PPG (Bis∼Methoxy PEG-13 PEG-438/PPG-110 SMDI Copolymer), a thermo-sensitive polymer capable of increase the viscosity and therefore the adhesiveness with increasing temperature.

Examples of additional excipients which can be used in said compositions are xanthan gum, carboxymethyl cellulose, silicone, petrolatum, white petrolatum, magnesium stearate, maltodextrin, mannitol, starch, glucose, glycerin, propylene glycol and the like.

The composition according to the invention may be in any form deemed appropriate by the skilled in the field to be administered via the respiratory tract.

The composition according to the invention comprising the association of the strain *S. salivarius* DSM 23307 and cysteine can be used as a valid alternative to the prior art compositions used to compete in the colonization of the respiratory mucosa by the bacteria responsible for naso-pharyngo-tubal infections on which exert their action.

With the expression "administration via the respiratory tract" in the present description it should be intended intranasal administration or inhalation.

According to the invention examples of suitable pharmaceutical forms are: solutions, suspensions, emulsions, sprays, drops, ointment, gel.

Preferably, the compositions is formulated to be administrated via the respiratory tract by means of a nebuliser sprays with or without propellants.

Dosages and timing of intake should be determined by the physician based on the general health condition of the recipient of the treatment and the severity of the clinical conditions.

Generally, 2 sprays into each nostril or mouth daily for 7 days a month for 3/5 months are recommended.

For this purpose the invention provides the use of an atomizer spray bottle of 10-15 ml volume, containing the final composition ready for use comprising the association of the bacterial strain *S. salivarius* DSM 23307 and cysteine, both reconstituted in the suitable solvent as above described, which allows immediate administration by the end user within a time span of 5 days, wherein the bacterium maintains its vitality.

In a preferred embodiment of the invention it is provided a kit comprising a 10-15 ml sterile vial containing the sterile isotonic saline solution, buffered at pH 7.0, comprising PEG/PPG copolymer, and flavorings; a heat-sealed sachet/stick pack format containing the lyophilized bacterial strain *S. salivarius* DSM 23307; a heat-sealed sachet/stick pack format containing lyophilized cysteine; a sterilized sprays nebulizer with protective cap.

Part of the kit are all the elements necessary for treatment, along with instructions for product assembly, use and storage of the product.

In another embodiment of the invention cysteine and bacterial strain are lyophilized and supplied in the same heat-sealed sachet/stick pack format. Therefore, in this case the kit includes a sterile vial from 10-15 ml containing the isotonic saline solution buffered at pH 7.0 sterile copolymer comprising PEG/PPG, and flavorings; a heat-sealed sachet format stick containing the mixture of the bacterial strain *S. salivarius* DSM 23307 and cysteine lyophilized; a sterile nebulizer sprays with protective cap.

The invention also provides a method of treatment consisting in the at least competing in the colonization of the naso-pharyngo-tubal district by microorganisms responsible for the onset of bacterial or viral diseases. The treatment is consisting in the administration of an effective amount of the composition above described according to the invention to a subject, or patient, in the need thereof. The treatment is addressed to determine the healing of the subject, or patient undergoing treatment, or reducing the severity of symptoms related to the infection.

In a further embodiment, the method of treatment may include a step of pre-treatment of the patient to reduce the population of the normal microbioma including the particular pathogenic bacterial strain which is to be inhibited. Such pre-treatment can be carried out by antibiotic administration. An example of suitable antibiotics for this purpose is amoxicillin + clavulanic acid. Clorexidina, erythromycin, penicillin and other antibiotic agents known to the expert of the field can be used as well.

In order to evaluate the efficacy and tolerability of the composition according to the invention a randomized double-blind, placebo-controlled study has been carried out.

The collected clinical results are now being analyzed and evaluated.

Further aspects of the invention will now be illustrated by way of example and not for limiting purposes in the examples given below.

### EXAMPLES

Example 1: Formulation of the isotonic saline solution wherein the lyophilized microorganism and cysteine are reconstituted.
a) Solvent formulation:

| **Ingredient** | **Amount % w/w** |
|---|---|
| Bis-methoxy PEG13 PEG438/PPG 110 SMDI Copolymer | 0,01-10 |
| Monobasic Sodium phosphate | q.s. to pH 7,00 and 300 mOsm |
| Dibasic Sodium phosphate | |
| Sodium chloride | |
| Flavourings | 0,0001-0,50 % w/w |
| Deionized water | q.s. to 100 % w/w |

Example 2: Formulation of freeze-dried powder comprising the bacterium antagonist of pathogenic flora of the nasal mucosa and cysteine
b) Lyophilized powder formulation:

| **Ingredient** | **Amount** | **Stick weight** |
|---|---|---|
| *Streptococcus salivarius* DSM 23307 | 3x10⁵-3x10¹⁴ cfu/g | 1 to 10 g per Stick |
| Cysteine hydrochloride | 0,0005-10,00 %w/w | |

## Claims

1. Association constituted by the bacterial strain *Streptococcus salivarius DSM 23307* and cysteine as active ingredient for the use in the treatment of upper respiratory tract inflammations and infections.

2. Composition comprising the association of bacterial *strain Streptococcus salivarius DSM 23307* and cysteine.

3. Composition according to claim 2 **characterized in that** the lyophilized bacterial strain *Streptococcus salivarius DSM 23307* is dissolved in a saline isotonic, solvent, buffered at pH 7.0, comprising bis-Methoxy PEG13 PEG438/PPG 110 SMDI copolymer and flavorings, having osmolarity of 300 mOsm.

4. Composition according to claim 2 wherein the bacterial strain *Streptococcus salivarius DSM 23307* and cysteine are in the form of lyophilized mixture dissolved in a saline isotonic solvent, buffered at pH 7.0, comprising bis-Methoxy PEG13 PEG438/PPG 110 SMDI copolymer and flavorings, having osmolarity of 300 mOsm.

5. Composition according to claims 2 to 4 wherein the final reconstituted bacterial suspension contains *S. salivarius DSM 23307* in an amount ranging between 3 x 10⁵ and 3 x 10¹⁴ cfu (colony forming units) per gram of composition in a volume of 10-50 ml.

6. Composition according to claim 5 wherein the final reconstituted bacterial suspension contains *S. salivarius DSM 23307* in an amount equal to 1-5 x 10¹¹ cfu per gram of composition in a volume of 10-15 ml.

7. Composition according to claims 2 to 6 further comprising one or more carriers and vehicles, diluents and/or pharmaceutically acceptable excipients, and further active ingredients.

8. Composition according to claim 7 wherein the carriers and vehicles are suitable to increase the bioavailability, stability and tolerability of the microorganism.

9. Composition according to claims 2 to 8 **characterized in that** from 1 to 10 g of *Streptococcus salivarius DSM 23307* (3x10⁵-3x 10¹⁴ cfu/g) are dissolved in 10-15 ml of saline isotonic solution having the formulation:
| | |
|---|---|
| bis-Methoxy PEG13 PEG438/PPG 110 SMDI copolymer | 0,01-10% w/w |
| Monobasic sodium phosphate | q.s. to pH 7,00 and 300 mOsm |
| Dibasic sodium phosphate | |
| Sodium chloride | |
| Flavoring | 0,0001- 0,50 % w/w |
| Deionized water | q.s. a 100 % w/w |

10. Composition according to claims 2 to 9 in a form suitable for administration via the respiratory tract, for intranasal administration, for administration by inhalation.

11. Composition according to claim 10 in the form of solution, suspension, emulsion, spray, drops, ointment, gel.

12. Kit comprising:
i) 10-15 ml sterile vial containing sterile saline isotonic buffered at pH 7.0 solution comprising PEG13-methoxy PEG438/PPG 110 SMDI copolymer, and flavorings;
ii) heat-sealed sachet/stick pack format containing the freeze-dried bacterial strain *S. salivarius DSM 23307;*
iii) heat-sealed sachet/stick pack format containing freeze-dried cysteine;
iv) sterilized spray atomizer with protective cap;
v) information leaflet with instructions for assembly, use and storage of the product.

13. Kit comprising:
i) 10-15 ml sterile vial containing sterile saline isotonic buffered at pH 7.0 solution comprising PEG13-methoxy PEG438/PPG 110 SMDI copolymer, and flavorings;
ii) heat-sealed sachet/stick pack format containing the mixture of freeze-dried bacterial strain *S. salivarius DSM 23307* and cysteine;
iii) sterilized spray atomizer with protective cap;
iv) information leaflet with instructions for assembly, use and storage of the product.

14. A composition comprising the association of bacterial strain *Streptococcus salivarius DSM 23307* and cysteine for medical use.

15. A composition comprising the association of bacterial strain *Streptococcus salivarius DSM 23307* and cysteine for use in the treatment of upper respiratory tract inflammations and infections.
